**Europäisches Patentamt**

**European Patent Office**

**Office européen des brevets**

(19)

(11) Veröffentlichungsnummer: **0 262 354 B1**

(12) **EUROPÄISCHE PATENTSCHRIFT**

(45) Veröffentlichungstag der Patentschrift:
02.01.91 Patentblatt 91/01

(51) Int. Cl.$^5$: **A61C 8/00, A61F 2/02**

(21) Anmeldenummer: **87111618.2**

(22) Anmeldetag: **11.08.87**

(54) Implantat aus Titan mit drahtförmigen Oberflächenstrukturen.

(30) Priorität: 02.09.86 DE 3629813

(43) Veröffentlichungstag der Anmeldung:
06.04.88 Patentblatt 88/14

(45) Bekanntmachung des Hinweises auf die
Patenterteilung:
02.01.91 Patentblatt 91/01

(84) Benannte Vertragsstaaten:
AT CH ES FR GB IT LI SE

(56) Entgegenhaltungen:
DE-A- 2 500 914
FR-A- 2 178 969
FR-A- 2 429 589
US-A- 3 807 005
US-A- 4 199 864
US-A- 4 492 577

(73) Patentinhaber: **Friedrichsfeld GmbH Keramik-
und Kunststoffwerke
Steinzeugstrasse 50
D-6800 Mannheim 71 (DE)**

(72) Erfinder: **Breme, Jürgen, Dr.-Ing.
H.-Löns-Strasse 15
D-8523 Baiersdorf (DE)**
Erfinder: **Heimke, Günther, Dr.
Zinkgräfstrasse 62
D-6940 Weinheim (DE)**
Erfinder: **Schulte, Willi, Prof. Dr.
Heuberger Tor Weg 23
D-7400 Tübingen (DE)**

## Beschreibung

Die Erfindung betrifft ein Verfahren zum Herstellen von Implantaten aus Titan mit drahtförmigen Oberflächenstrukturen.

Implantate mit drahtförmigen Oberflächenstrukturen dienen in der Zahnheilkunde z.B. als Ersatz von ganzen Zahnwurzeln oder in der Orthopädie z.B. als Hüftgelenkersatz. Dabei sind stets verschiedene Teile der jeweiligen Implantate zu unterscheiden : Einmal er Implantatteil, der der Verankerung des Implantats im jeweiligen knöchernen Bett dient, zum anderen der Implantatteil, der die jeweils zu ersetzende Funktion ausübt. Häufig gibt es dazwischen noch einen weiteren Implantatabschnitt, der den Verankerungsteil mit dem Funktionsteil verbindet. Im Falle eines Zahnimplantates wird der Verankerungsteil in den Kieferknochen eingesetzt, der Funktionsteil ist die jeweilige Zahnkrone oder die Brücke, die von dem Implantat getragen wird. Als Verbindungsteil kann in diesem Fall derjenige Abschnitt des Implantats angesehen werden, der durch die Gingiva hindurch die Verbindung vom Verankerungsteil zum Funktionsteil herstellt. Im Falle des Schaftes einer Hüftgelenkprothese dient der eigentliche Prothesenschaft der Verankerung, der Funktionsteil ist in diesem Fall der künstliche Hüftgelenkkopf und als Verbindungsteil ist der Halsbereich der Prothese zwischen dem Kopf und dem Schaft anzusehen. Derartige Implantate haben natürlich die Aufgabe die Funktionen des Knochengewebes, als dessen Ersatz sie eingebracht worden sind, möglichst lange und zuverlässig und für den Patienten beschwerdefrei zu ersetzen.

Das hier vorgeschlagene Verfahren beschränkt sich auf die Verankerungsteile derartiger Implantate.

Für die Verankerungsteile derartiger Implantate sind bereits drahtförmige oder drahtähnliche Oberflächenstrukturen vorgeschlagen worden. (P. Ducheyne, P. de Meester und E. Aernoudt, "Isostatically compacted metal fibre porous coatings for bone ingrowth", Powder Metallurgy International, Vol. 11, No. 3, 1979, S. 115 - 119, siehe besonders Figuren 5, 6, 7 und 9). Auch die Strukturen, die in der DE-OS 34 34 309 beschrieben worden sind, können als drahtähnlich betrachtet werden, wenn auch mit außerordentlich ungleichmäßiger Konfiguration. - All diesen bisherigen Vorschlägen ist aber gemeinsam, daß es sich bei den Grundkörpern dieser Implantate sowohl als auch bei den drahtförmigen Oberflächenstrukturen nicht um Titanimplantate handelt. Diese bisher bekannten Implantate bestehen vielmehr entweder im wesentlichen aus Edelstählen oder aus Legierungen aus dem Zusammensetzungsbereich Kobalt-Chrom-Molybdän. Dies Legierungen geben jedoch im Körpermilieu Bestandteile an ihre Umgebung ab, im allgemeinen in Form von Metallionen. Es ist bekannt, daß derartige Metallionen die Aktivitäten der knochenbildenden Zellen in der Umgebung derartiger Implantate beeinflussen. Es kommt daher im allgemeinen nicht zu der erwünschten Knochenneubildung im unmittelbaren Kontakt mit den Oberflächen der Drähte und Implantatkörper.

Ein derartiger erwünschter Knochenkontakt zu metallischen Implantatoberflächen wurde jedoch bereits an Titanimplantaten beobachtet. Es wurde sogar gefunden, daß Knochengewebe an Titanoberflächen wie bei einer Klebung anhaftet. Bei den Versuchen, derartige Verbunde zwischen knochengewebe und Titanimplantaten gewaltsam zu trennen, blied häufig Knochenmaterial auf der Titanoberfläche haften und der Abriß erfolgte innerhalb des Knochengewebes.

In der FR-A-2429589 ist der Schatf der Fermurkomponente einer Hüftendoprothese aus Titan mit aufgeschweissten Titandrähten mit einem Durchmesser von weniger als 0,3 mm vorgeschlagen worden. Derartige Schäfte haben jedoch keinen Eingang in die klinische praxis gefunden. Das Offenhalten der für das Einwaschen von Knochengewebe notwendigen Freiräume ist bei derart filigranen Strukturen und der Notwendigkeit einer bereits initial festen Verankerung (Presssitz) nicht möglich.

Um das günstige Verhalten von Knochengewebe gegenüber Titanoberflächen zur weiteren Verbesserung der verankerung von Implantation in Knöcherner Umgebung nützen zu können, besteht daher einmal die Aufgabe, sicher, d.h. auch unter den Bedingungen einer initial festen Verankerung offen bleibende drahtförmige Oberflächenstrukturen auf Implantaten aus Titan einzuführen. Diese Aufgabe konnte aber bisher nicht gelöst werden, weil es giesstechnisch entweder nicht oder nur mit kommerziell nicht zu wertretendem Aufwand möglich war, drahtförmige Oberflächenstrukturen in den notwendigen Dimensionen bezüglich Drahtdurchmesser und Abmessungen der zwischen den Drähten verbliebenen Freiräume herzustellen. Es besteht also die weitere Aufgabe, ein Verfahen anzugeben, bei dem es mit technisch kommerziell vernünfigem Aufwand möglich ist, derartige drahtförmige Oberflächenstrukturen auf Titanimplantaten zu realisieren.

Die Aufgabe wird durch das Verfahren gelöst, das in dem kennzeichnenden teil des Anspruchs 1 beschrieben wird. Weitere Einzelheiten des Verfahrens sind in den unteransprüchen erwähnt.

Die grundkörper der verankerungsteile der Implantate bestehen erfindungsgemäß aus im wesentlichen dichtem Titan. Sie haben daher auch eine im wesentlichen dichte Oberfläche, wie sie bei der bießtechnischen und unformtechnischen Herstellung derartiger Implantate üblich ist. Die Oberflächen können aber zusätzlich noch Strukturen wie Nuten, Lakunen oder dergleichen aufweisen. Die Grundkörper der Implantate können darüber hinaus auch Bohrungen oder Zapfen aufweisen, wie sie insbesondere zur Ver-

bindung des Grundkörpers zum Übergangsteil oder zum Funktionsteil notwendig sind. Die Grundkörper können auch die Form von Hohlzylindern haben. Unter Titan sollen im Rahmen dieses Patentes neben Reintitan, technisch reinem Titan und deren Varianten auch Titanlegierungen verstanden werden, die neben Titan auch noch Zusätze von Aluminium, Eisen, Vanadium und ähnlichen, in Mengen, wie sie für die Erzielung von Festigkeitserhöhungen notwendig sind, enthalten.

Für das Herstellen eines dichten Geflechtes aus Titan- und Eisendrähten kommen alle für die Herstellung von Metallgeweben üblichen Verfahren in Frage. Auch das Flechten kann in seinen unterschiedlichen Varianten benutzt werden. Da die durchgehenden Offenräume in diesen Geflechten durch das spätere Herauslösen der Eisenkomponenten hergestellt werden, bestimmt der Durchmesser der Eisenkomponente im wesentlichen die Abmessungen dieser Offenräume. Neben er Größe der Offenräume durch Wahl der Eisendrahtdurchmesser ist auch die Dichte der Offenräume bei dem hier angegebenen Verfahren in weiten Grenzen dadurch variierbar, daß die Durchmesser der Eisendrähte und der Titandrähte verschieden gewählt werden und daß das Verhältnis der Anzahl der Titandrähte zur Anzahl der Eisendrähte je Flächen- oder Volumeneinheit geändert wird. Das hier angegebene Verfahren gestattet es daher den unterschiedlichsten medizinischen Anforderungen für die Verankerung von Implantaten in verschiedenen Teilen des Skelettes gerecht zu werden. Eine verhältnismäßig gleichmäßige Verteilung von Offen- zu durch Titan erfülltem Raum innerhalb der drahtförmigen Oberflächenstrukturen mit einem für den Bereich der Zahnimplantate günstigen Abmessungsverhältnis ergib sich bei Wahl der Drahtdurchmesser im Bereich von 0,5 mm. - Eine Erhöhung der Dichte innerhalb des Geflechtes läßt sich durch Pressen oder Strecken erreichen.

Das dichte oder ggf. nachverdichtete Geflecht wird auf den Grundkörper durch Auflegen oder Aufwickeln aufgebracht. Es kann dabei durch zusätzliche Hilfsmittel temporär in Position gehalten werden. Hierzu kann auch die für das endgültige Befestigen des Geflechtes auf dem Grundkörper ggf. benötigte Form oder das ggf. dafür vorgesehene Werkzeug benutzt werden.

Die endgültige Befestigung des Geflechtes auf dem Grundkörper erfolgt durch Verschweißen. Hierfür kommen z.B. alle Preßschweißverfahren in Frage, so auch das Heißisostatischpressen.

Die Herstellung des für das Einwachsen des Gewebes und damit die Verankerung des Implantats im Knochenmilieu notwendigen Offenraumes in den drahtförmigen Oberflächenstrukturen erfolgt durch selektives Auflösen der Eisendrähte. Dies kann durch Eintauchen des entsprechend vorbereiteten Implantats in einer oxidierende Säure, gegen die Titan resistent ist, z.B. Salpetersäure, bewirkt werden. Diese Technik ist bereits bekannt.

Erste tierexperimentelle Untersuchungen an nach dem hier beschriebenen Verfahren hergestellten Probekörpern haben gezeigt, daß das erwartete Einwachsen des Knochengewebes in die Offenräume der Oberflächenstrukturen in der erwartenden Weise erfolgt und sich ein enger Knochenkontakt einstellt.

## Ansprüche

1. Verfahren zum Herstellen von Implantaten, die aus einem grundkörper aus Titan mit darauf aufgeschweissten, drahtförmigen Oberflächenstrukturen bestehen, gekennzeichnet durch folgende Arbeitsgänge :

1. Herstellen eines dichten Geflechtes aus Titan- und Eisendrähten, so dass die Eisendrähte im wesentlichen die späteren offenräume der drahtförmigen Oberflächenstrukturen einnehmen, und,

2. nach dem Aufschweissen dieses geflechtes auf dem Grundkörper, selektives Auflösen des Eisendrahtes durch eine oxidierende Säure, gegen die Titan resistent ist.

2. Verfahren nach Anspruch 1, dadurch gekennzeichnet, daß zum Herstellen des Geflechtes Drähte mit einem Durchmesser von 0,5 mm benutzt werden

3. Verfahren nach Anspruch 1, dadurch gekennzeichnet, daß das Geflecht aus Zöpfen zusammengesetzt wird für die je zwei Titandrähte und ein Eisendraht zu einem "Zopf" geflochten werden.

4. Verfahren nach Anspruch 1, dadurch gekennzeichnet, daß das Geflecht aus einem Netz aus Titan- und Eisendrähten besteht.

5. Verfahren nach Anspruch 1, dadurch gekennzeichnet, daß das Verbinden des Geflechtes mit dem Grundkörper durch ein Preßschweißverfahren bewirkt wird.

6. Verfahren nach Anspruch 1, dadurch gekennzeichnet, daß als oxidierende Säure zum Herauslösen der Eisendrähte Salpetersäure benutzt wird.

## Claims

1. Procedure for the manufacture of implants which consist of a main body of titanium with surface structures composed of wires welded onto said main body, characterized by the following manufacturing steps :

1. Mating of a dense mesh consisting of titanium and iron wires in a manner so that the iron wires occupy the intended open spaces of said surface structures, and,

2. after welding said mesh onto said main body, selectively dissolving the iron wires by an oxidizing acid in which titanium is unsoluble.

2. Procedure according to claim 1, characterized by the use of wires with a diameter of 0.5 mm for the making of said mesh.

3. Procedure according to claim in characterized by the composition of said mesh of plaits for which two titanium wires and one iron wire are braided into one "plait".

4. Procedure according to claim in characterized in that the mesh consists of a net composed of titanium and iron wires.

5. Prcocedure according to claim in characterized by the joining of said mesh to said main body by pressure welding.

6. Procedure according to claim in characterized by the use of nitric acid as said oxidizing acid for the dissolution of the iron wires.

**Revendications**

1. La procédure pour la fabrication d'implants qui consistent en un corps principal en titane avec des structures qui recouvrent la surface en forme de filament et qui sont brasées sur ce corps principal est caractérisée par des phases de fabrication suivantes:

1. La fabrication d'un treillis dense consiste en un réseau de fils de titane et fils de fer, constitué de telle manière que les fils de fer occupent présentement les futur mailles de cette surface, et,

2. après la brasure de ce treillis sur le corps principal, dissolution selective du fil de fer par un acide oxydant auquel le titane résiste.

2. Procédure selon exigence 1, caractérisée par l'utilisation de fils avec un diamètre de 0,5 mm pour la fabrication du treillis.

3. Procédure selon exigence 1, caractérisée par le fait que le treillis est composé de tresses composées chacunes avec deux fils de titane et un fil de fer.

4. Procédure selon exigence 1, caractérisée par le fait que le treillis consiste en un réseau de fils de titane et de fils de fer.

5. Procédure selon exigence 1, caractérisée par le fait que la jonction du treillis avec le corps principal est produite par un procédé de brasure sous pression.

6. Procédure selon exigence 1, caractérisée par l'utilisation de l'acide nitrique comme acide oxydant pour la dissolution des fils de fer.